# EUROPEAN PATENT SPECIFICATION

(11) **EP 2 109 623 B1**
(45) Date of publication and mention of the grant of the patent: **12.10.2011**
(21) Application number: 07734628.6
(22) Date of filing: 22.05.2007
(51) Int. Cl.: C07K 16/24

(54) **TREATMENT OF CANCER WITH ANTI-IL-1 ANTIBODIES**
KREBSBEHANDLUNG MIT ANTI-IL-1-ANTIKÖRPERN
TRAITEMENT DU CANCER PAR DES ANTICORPS ANTI-IL-1

(30) Priority: 22.05.2006 US 802166 P
(43) Date of publication of application: 21.10.2009
(73) Proprietor: XBiotech, Inc, Vancouver, BC V6E 2E9 (CA)
(72) Inventor: SIMARD, John, Austin, TX 78733 (US)
(74) Representative: Tombling, Adrian George
(86) International application number: PCT/IB2007/001320
(87) International publication number: WO 2007/135546

(56) References cited:
- US-A- 5 959 085
- US-A1- 2003 026 806
- ITO R ET AL: "Interleukin 1 alpha acts as an autocrine growth stimulator for human gastric carcinoma cells." CANCER RESEARCH 1 SEP 1993, vol. 53, no. 17, 1 September 1993 (1993-09-01), pages 4102-4106, XP002459296 ISSN: 0008-5472
- SHIRAKAWA F ET AL: "Autocrine stimulation of interleukin 1 alpha in the growth of adult human T-cell leukemia cells." CANCER RESEARCH 1 MAR 1989, vol. 49, no. 5, 1 March 1989 (1989-03-01), pages 1143-1147, XP002459297 ISSN: 0008-5472
- APTE ET AL: "Effects of micro-environment- and malignant cell-derived interleukin-1 in carcinogenesis, tumour invasiveness and tumour-host interactions" EUROPEAN JOURNAL OF CANCER, PERGAMON PRESS, OXFORD, GB, vol. 42, no. 6, April 2006 (2006-04), pages 751-759, XP005412577 ISSN: 0959-8049
- DINARELLO C A ET AL: "THE ROLE OF INTERLEUKIN-1 IN DISEASE" NEW ENGLAND JOURNAL OF MEDICINE, THE, MASSACHUSETTS MEDICAL SOCIETY, WALTHAM, MA, US, vol. 328, no. 2, 1993, pages 106-113, XP009076400 ISSN: 0028-4793

## Description

### BACKGROUND OF THE INVENTION

Cancer generally kills by invading adjacent tissue structures, disrupting the physiology of critical organs. The process of metastasis has been found to be concurrent with de-differentiation of primary tumor lesions. A corollary of tumor de-differentiation is tumor heterogeneity. The triad of de-differentiation, heterogeneity and metastasis makes for a deadly mix. Once cancer has become both metastatic and heterogeneous, the possibility of complete anti-tumor treatment is remote. The longstanding hope has been to identify some common element of metastatic tumors, a crucial feature that is retained during outgrowth and de-differentiation in even the most heterogeneous tumors, a feature that is so intrinsic to the process of metastasis that it is present in virtually all tumor cells regardless of origin or tropism. With the identification of such crucial elements, the notion of treating advanced, disseminated disease may have a basis in reality.

### BRIEF DESCRIPTION OF THE FIGURE

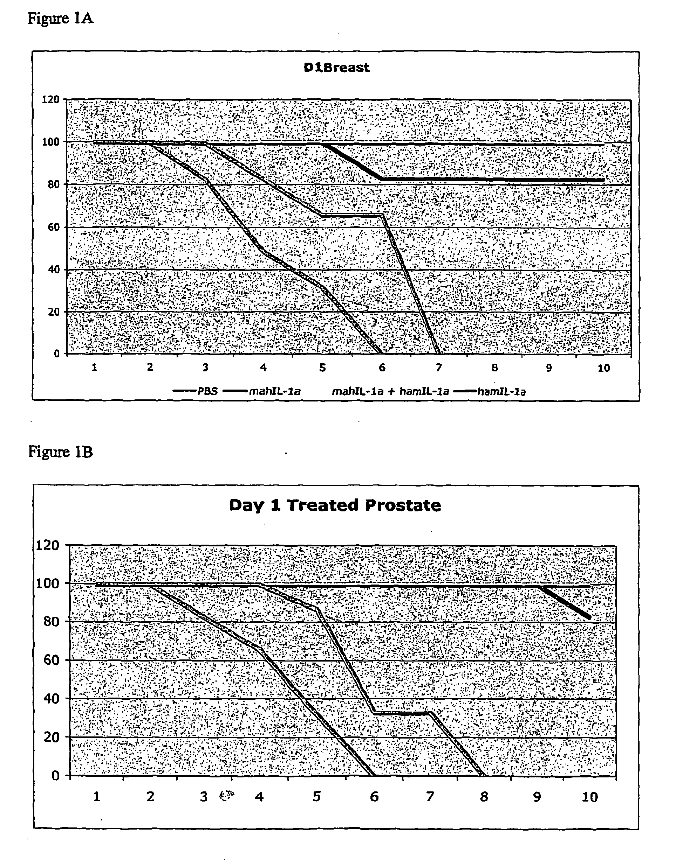

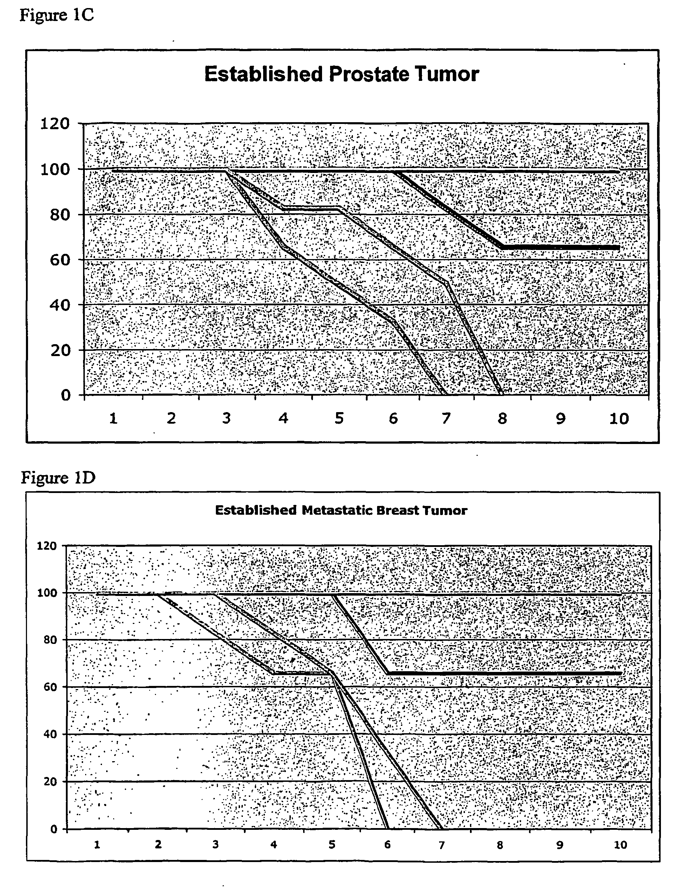

Figure 1. Graphs showing tumor responses in nude mice with human tumor xenotransplants after treatment with anti-IL-1α antibodies. Mice are treated with either mouse-anti-human anti-IL-1a monoclonal antibody (ₘaₕIL-1α) or hamster anti-mouse IL-1a monoclonal antibody (ₕaₘIL-1α) or both (ₘaₕIL-1α + ₕaₘIL-1α). Mice are given 5mg/kg doses of each antibody twice weekly starting on day of xenotransplant (Day 1 Tumor) or after establishment of metastatic disease (Established Tumor). Mice are sacrificed when carrying considerable tumor burden and in obvious discomfort. Figure 1A, prostate tumor, day 1; Figure 1B, breast tumor, day 1; Figure 1C, established prostate tumor; Figure 1D, established breast tumor.

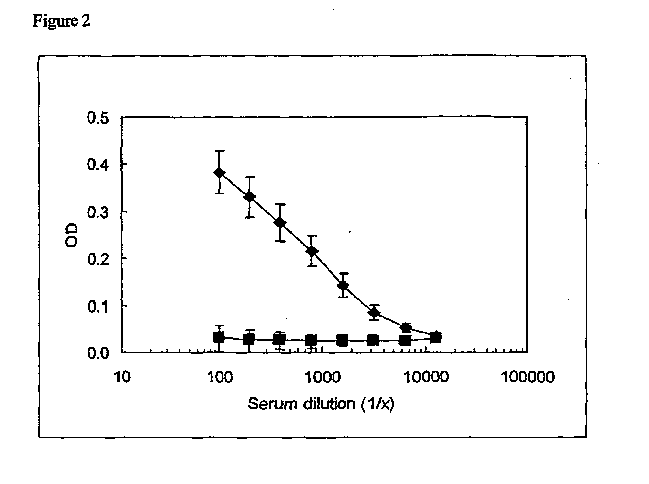

Figure 2. Anti-IL-1α autoantibody formation on day 56 in C57BL/6 mice after three subcutaneous injections with IL-1α-PPD conjugate in alum (◆). Control mice immunized with PPD in alum only (■).

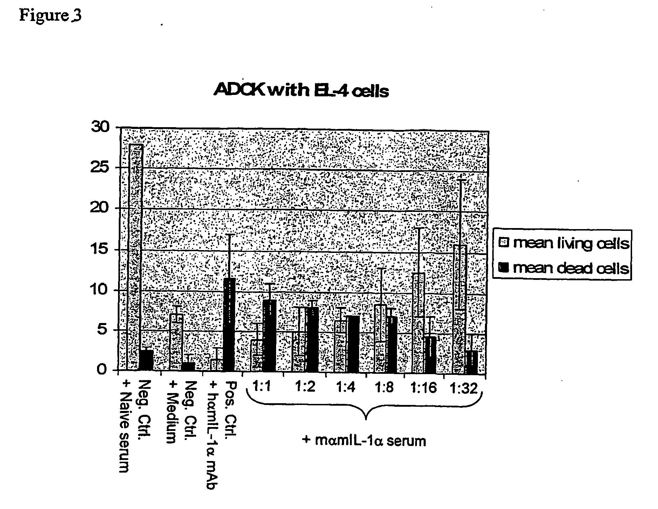

Figure 3. Antibody-dependent complement-mediated killing of EL-4 cells. EL-4 cells were incubated with serial dilutions of mouse anti-mouse IL-1α polyclonal antiserum. The ratio of killed cells to viable cells is proportional to the serum concentration. A human anti-mouse IL-1α monoclonal antibody was used as a positive control. Incubation with naïve murine serum or with culture medium alone served as the two negative controls.

### DETAILED DESCRIPTION OF THE INVENTION

Targeting IL-1α with an antibody can be used as a cancer treatment. In particular, anti-IL-1α antibody can inhibit metastatic potential of tumors through interruption of the physiological role tumor-derived IL-1α plays in tumor metastasis. Moreover, because IL-1α is expressed by tumors, an antibody targeting IL-1α can cause direct tumor cytotoxicity through antibody directed cellular cytotoxicity (commonly referred to as ADCC).

It is highly unexpected that interleukin-1 alpha (IL-1α) could be a target for cancer therapy. To understand this requires a brief review of the history of the so called interleukin-1 system. The IL-1 system includes IL-1α, interleukin-1 beta (IL-1β), interleukin-1 receptor antagonist (IL-1rα), and interleukin-1 receptor 1 (IL-1R1). After almost three decades since the discovery of IL-1α and IL-1β, there has been little progress made in distinguishing separate biological roles for the two gene products. The inability to elucidate the independent biological functions of these two cytokines is evidenced by the common reference in the scientific literature simply to interleukin-1, which has for decades been the nomenclature that collectively refers to IL-1α and/or IL-1β. This failure to distinguish these two cytokines is as unique as it is peculiar, considering the rather clear differences between IL-1α and IL-1β: they do not share significant protein sequence homology; they are under different transcriptional regulation, resulting in temporal and spatial separation of expression; they are independently regulated through separate and individually complex post-translational processing machinery; they are subjected to unique and separate post-translation modifications; and they have different tissue distribution and are up-regulated in response to different stimuli. Moreover, IL-1α is membrane anchored via a lipid tail and has lectin-like binding activity, whereas IL-1β is a secreted protein.

Considering the differences between these cytokines, it is worth understanding why IL-1α and IL-1β should have been collectively referred as interleukin-1. Firstly, the early assumption was that the two gene products represented only a single biological activity, or to perhaps put a more fine point to it, that IL-1α had little notable biological function. This disregarding of IL-1α resulted from the fact that there was no known secretory mechanism for the cytokine, no transmembrane sequence that would enable integration in the membrane, and no encoded signal sequence for translocation to secretory vesicles. IL-1α was thought to be contained in the cytoplasm, and a role as an intracellular signaling molecule suggested little relevance as a true cytokine. On the other hand, a post translational processing and secretory pathway was quickly established for IL-1β. In fact, the only relevance of IL-1α in the so called interleukin-1 system, seemed to be that it was shown to induce signaling through the IL-1 receptor-1, which was found to be induce signaling in response to IL-1α and IL-1β. Because there was no mechanism for secretion, it was postulated that IL-1α might only effect a physiological role when it was released from the cytoplasm of dead cells. But the failure to find significant levels of IL-1α in sera or tissues under almost any circumstances seemed to minimize the possible importance of IL-1α.

It is thus quite unexpected that treatment of animals with an anti-IL-1α antibody can protect the animals from aggressive forms of cancer. Similarly, immunization of animals to induce anti-IL-1α antibody titer can protect the animals from tumors. The mechanism of action is not yet clear and may involve one or more combinations of a neutralization of host pro-tumor IL-1α production, neutralization of tumor IL-1α production or direct cyto-toxicity of tumor via antibody directed cellular (ADCC) or complement mediated killing (ADCK).

Patients who can be treated according to the invention include both humans and non-human mammals, such as companion animals, laboratory animals, animal models, etc. (*e.g*., cats, dogs, sheep, pigs, goats).

"Antibodies" as used herein includes intact polyclonal or monoclonal immunoglobulin molecules; immunoglobulin fragments, such as monomeric and dimeric Fab, F(ab')₂, scFv, and Fv; and non-naturally occurring molecules such as diabodies, minibodies, Kappa bodies, Janusins, and the like. Antibodies useful in therapeutic methods of the invention comprise an IL-1α binding site and specifically bind to IL-1α. "IL-1α binding sites" as used herein include IL-1α binding sites which naturally occur in the variable portion of antibodies. IL-1α binding sites also include binding sites which differ from naturally occurring IL-1α binding sites by between 1 and 15 (*e.g*., 1, 2, 3, 4, 5, 6, 7, 8, 9, 10, 11, 12, 13, 14, or 15) conservative amino acid substitutions and which specifically bind to IL-1α. Typically, an antibody which specifically binds to IL-1α provides a detection signal at least 10-, 20-, or 100-fold higher than a detection signal provided with a non-IL-1α antigen when used in an immunochemical assay. Preferably, antibodies which specifically bind to IL-1α do not detect other proteins in immunochemical assays and can immunoprecipitate IL-1α from solution.

Polyclonal antibodies can be obtained by immunizing an appropriate host with IL-1α using well-known methods. However, monoclonal antibodies are preferred. Monoclonal antibodies (*e.g*., full-length, scFv, Fv) can be prepared using any technique that provides for the production of antibody molecules by continuous cell lines in culture. These techniques include, but are not limited to, the hybridoma technique, the human B-cell hybridoma technique, and the EBV-hybridoma technique. *See* Roberge et al., Science 269, 202-204, 1995; Kohler et al., Nature 256, 495-497, 1985; Kozbor et al., J. Immunol. Methods 81, 31-42, 1985; Cote et al., Proc. Natl. Acad Sci. 80, 2026-2030, 1983; and Shimamoto et al., Biologicals, 2005 Sep;33(3):169-74. Single chain antibodies can be generated by chain shuffling from random combinatorial libraries. Takeda et al., Nature 314, 452-454, 1985.

Single-chain antibodies also can be constructed using a DNA amplification method, such as PCR, using hybridoma cDNA as a template. Single-chain antibodies can be mono- or bispecific, and can be bivalent or tetravalent Construction of tetravalent, bispecific single-chain antibodies is well known in the art. A nucleotide sequence encoding a single-chain antibody can be constructed using manual or automated nucleotide synthesis, cloned into an expression construct using standard recombinant DNA methods, and introduced into a cell to express the coding sequence. Alternatively, single-chain antibodies can be produced directly using, for example, filamentous phage technology. Burton et al., Proc. Natl. Acad. Sci. 88, 11120-23, 1991; Verhaar et al., Int. J. Cancer 61, 497-501, 1995.

IL-1α antibodies useful in the invention can be purified from any cell which expresses the antibodies, including host cells which have been transfected with antibody-encoding nucleic acid molecules. The host cells are cultured under conditions suitable for expression of the antibodies. Appropriate host cells and culture conditions can be selected from the wide variety known in the art.

Purified antibodies are separated from other compounds that normally associate with the antibody in the cell, such as certain proteins, carbohydrates, or lipids. Purification methods include, but are not limited to, size exclusion chromatography, ammonium sulfate fractionation, ion exchange chromatography, affinity chromatography, and preparative gel electrophoresis. A preparation of purified antibodies is at least 80% pure; preferably, the preparations are 90%, 95%, or 99% pure. Purity of the preparations can be assessed by any means known in the art, such as SDS-polyacrylamide gel electrophoresis. A preparation of purified antibodies of the invention can contain more than one type of antibody which specifically binds to IL-Ia.

Full-length polyclonal or monoclonal antibodies, however prepared, can be cleaved with standard techniques to obtain functional antibody fragments such as Fab or F(ab')2. *See* Cheung et al., Protein Expr. Purif. 32, 135-40, 2003. Binding proteins which are derived from immunoglobulins and which are multivalent and multispecific, such as the "diabodies" described in WO 94/13804 and Holliger et al., Proc. Natl. Acad Sci. USA 90, 6444-48, 1993; the "minibodies" described in Martin et al., EMBO J. 13, 5303-09, 1994; "Kappa bodies" described in Ill et al., Protein Eng. 10, 949-57, 1997; and "Janusins" (bispecific single chain molecules) described in Traunecker et al., EMBO J. 10, 3655 3659, 1991, and Traunecker et al., Int. J. Cancer Suppl. 7, 51-52, 1992, can be prepared.

Any IL-1α antibody useful in the invention also can be produced using chemical methods to synthesize its amino acid sequence, such as by direct peptide synthesis using solid-phase techniques (Merrifield, J. Am. Chem. Soc. 85, 2149-54, 1963; Roberge et al., Science 269, 202-04, 1995). Protein synthesis can be performed using manual techniques or by automation. Automated synthesis can be achieved, for example, using Applied Biosystems 431A Peptide Synthesizer (Perkin Elmer). Optionally, fragments of antibodies can be separately synthesized and combined using chemical methods to produce a full-length molecule. The newly synthesized molecules can be substantially purified by preparative high performance liquid chromatography (*e.g*., Creighton, PROTEINS: STRUCTURES AND MOLECULAR PRINCIPLES, WH Freeman and Co., New York, N.Y., 1983). The composition of a synthetic polypeptide can be confirmed by amino acid analysis or sequencing (*e.g*., using Edman degradation).

Those skilled in the art can use known injectable, physiologically acceptable sterile solutions to prepare suitable pharmaceutical compositions comprising antibodies of the invention. Aqueous isotonic solutions, such as saline or corresponding plasma protein solutions, are readily available and can be used to prepare ready-to-use solutions for parenteral injection or infusion. Pharmaceutical compositions can be stored as lyophylisates or dry preparations, which can be reconstituted with a known injectable solution before use. A pharmaceutical composition can be supplemented with known carrier substances or/and additives (*e.g*., serum albumin, dextrose, sodium bisulfite, EDTA, etc.). Pharmaceutical compositions of the invention typically comprise a pharmaceutically acceptable vehicle, such as an inert diluent.

Pharmaceutical compositions of the invention can be administered by different routes known to those skilled in the art. For systemic application, the intravenous, intravascular, intramuscular, intraarterial, intraperitoneal, oral, intranodal, or intrathecal routes can be used. More localized application can be effected subcutaneously, intracutaneously, intracardially, intralobally, intramedullarly, intrapulmonarily, or directly in or near the tissue to be treated. Depending on the desired duration and effectiveness of the treatment, compositions may be administered once or several times, for example on a daily basis for several days, weeks or months, and in different dosages.

The dosage will depend on age, condition, sex and extent of the disease in the patient and can vary from 0.25 mg/kg to about 50 mg/kg of patient body weight. Cancers which can be treated include, but are not limited to, blood cancers (*e.g*., leukemias, lymphomas) and cancers of solid tissues (*e.g*., bladder, bone, brain, breast, cervix, colon, esophagus, kidney, liver, lung, pancreas, prostate, stomach).

In one embodiment, the patient is immunized against IL-1α to induce IL-1α antibodies. Any methods of immunization known in the art can be used to achieve the desired antibody response (see below). In general, recombinant IL-1α can be used in a formula containing an adjuvant to achieve immunization; a nucleic acid sequence encoding IL-1α can be used to make a recombinant virus or organism which can be used to immunize; or recombinant IL-1α can be chemically linked to virus-like particles, which act as immunostimulatory complexes.

| ADJUVANT | EXAMPLE |
|---|---|
| Inorganic Salt | Aluminum hydroxide, calcium phosphate, beryllium hydroxide |
| Delivery systems | Incomplete Freund's adjuvant |
| Bacterial Products | Complete Freund's Adjuvant, BCG, plasmid |
| DNA | CpG motifs |
| Immune Stimulatory Complexes (ISCOMS) | Mixture of Quil A containing viral proteins |
| Cytokines | GM-CSF, IL-12, IL-1, IL-2 |
| Recombinant Virus | Influenza |
| Virus-like particle conjugate | 2/6 VLP containing bovine rotavirus VP2 and human rotavirus VP6 |
| Recombinant Bacteria | Attenuated Salmonella typhimurium |

The above disclosure generally describes the present invention. A more complete understanding can be obtained by reference to the following specific examples, which are provided for purposes of illustration only and are not intended to limit the scope of the invention.

### EXAMPLE 1

### Animals and tumor cells

Data are generated using Athymic nu/nu mice (8-10-weeks-old, NCI, Frederick, MD). Mice are injected subcutaneously in the flank with 5 x10⁶ tumor cells suspended in 200µl of DMEM. Since we expected that IL-1α might provide a general mechanism for tumor cell viability, we tested several tumor cells lines for inhibition with anti-IL-1α, injecting animals with tumor cells derived from either breast (MDA-MB-436 or MDA-MB-231, Nozaki et al. Biochem Biophy Res Comm 275, 60-62 (2000)) or prostate (PC-3, Chung et al. The Prostate 38:199-207 (1999); Singer CF et al. Clin Cancer Res. 2003 Oct 15;9(13):4877-83) lineages, which have previously been shown to express IL-1α.

Mice are injected with mouse anti-human IL-1α antibodies to antagonize tumor IL-1α production. Mice receive either 5mg/kg IgG1 monoclonal antibody (clone 364-3B3-14, BioLegend) or 5mg/kg IgG2a monoclonal antibody (Clone 1F3B3, ProMab), administered intraperitoneally twice per week, starting on the day of tumor implantation or after evidence of a primary tumor lesion of at least about 3mm³. Two other groups of mice receive the IgG1 monoclonal antibody or IgG2a monoclonal antibody as well as 5mg/kg of an anti-mouse anti-IL-1α monoclonal antibody (Hamster anti-mouse IL-1α is purchased from BD PharMingen (San Diego, Calif)), in order to neutralize endogenous IL-1α production.

Animals are kept alive for 56 days unless sacrificed earlier for humanitarian reasons, due to excessive tumor burden. Each week animal body weights are recorded and observable tumor volume is measured. Animals are sacrificed when there is evident tumor-related morbidity (weight loss, lethargy). Mice are sacrificed using a CO₂ chamber. Metastases are harvested and stored separately after thorough examination of abdominal and peritoneal cavities and major organs, including liver, lymph nodes, spleen and lungs. Aggregate tumor mass is calculated. Survival and tumor burden results are expressed as mean +1- SE.

### EXAMPLE 2

### Immunohistochemistry

Metastatic tumor specimens resected with surrounding tissue are taken from some animals for histological analysis. Formalin-fixed, paraffin-embedded tumor preparations are generated at time of sacrifice. Histological analysis is performed using both the anti-murine and anti-human IL-1α antibodies, as well anti-VEGF, anti-ICAM-1, anti-E-Selectin and anti-VCAM staining is performed.

### EXAMPLE 3

### PCR

RNA is extracted from each of the tumor cell lines and from tumor biopsies taken from mice with established subcutaneously transplanted tumors. Cells are analyzed using RT-PCR for IL-1α transcripts. Primers are designed to specifically identify human IL-1α, so that there is no confusion in tumor biopsy samples whether or not the IL-1α transcripts are derived from the tumor cells or from endogenous production. Additionally, IL-1α mouse specific primers are designed to identify endogenous IL-1α that might have been produced from infiltrating leukocytes or from surrounding tissue of the tumor microenvironment. Since IL-1α from either source may be important in creating a favorable tumor microenvironment.

Total RNA is isolated from tumor samples with Trizol (Gibco/BRL Life Technologies, Rockville, MD, USA) as directed by the manufacturer. Contaminating DNA is removed with RNase free DNAse. One µg of DNAse treated total RNA (or water as a negative control) is incubated with 1µg oligo dT primer at 95°C for 3 min and then 68°C for 10 min. Eight µl of 5x buffer, 4µl DTT (0.1M), 2µl of dNTP (10mM), 1µl RNase inhibitor, and 1µl superscript reverse transcriptase are added to each reaction according to the method of Lee et al. (Journal of Orthopaedic Research, 21(2003) 62-72).

### EXAMPLE 4

### aIL-1α Antibody Reduces Metastatic Incidence

Nu/nu mice bearing established metastatic tumors are treated twice weekly with intraperitoneal injections of PBS, 5mg/kg of ₘaₕIL-1α b, or 5mg/kg of ₕaₘIL-1α b together with 5mg/kg of ₕaₘIL-1α b. Two groups of tumor bearing mice are used, those injected subcutaneously with 5x10⁶ MDA-MB-436 or PC-3 tumor cells. Antibody is administered twice weekly, starting either on the day of subcutaneous injection of tumor cells or after tumor growth of 3mm³. See Table 1.

**Table 1. Description of Experimental Design and Animal Numbers**

| | PBS | ₘaₕIL-1α+ₕaₘIL-1α | ₘaₕIL-1α | ₕaₘIL-1α |
|---|---|---|---|---|
| D1MDA-MB436 | 6 | 6 | 6 | 6 |
| D1PC-3 | 6 | 6 | 6 | 6 |
| EstMDA-MB436 | 6 | 6 | 6 | 6 |
| EstPC-3 | 6 | 6 | 6 | 6 |

Visible tumor colonies are counted on the organs at the time of sacrifice. The number of surface liver metastases is determined by inspection of the tissue to visualize tumor foci. The number metastatic foci on the diaphragm, intestine and peritoneal wall and lymph nodes is determined in a similar fashion.

In the breast tumor models visible lymph node, lung and liver metastases are reduced by treatment with either ₙaₕIL-1α or by combination treatment with ₘaₕIL-1α b + ₕaₘIL-1α b. One hundred percent of the control treated mice developed ascites in contrast to ascites formation in only 10% of anti-IL-1α treated mice. Similar observations are made with the prostate tumor model, where mice receiving either ₘaₕIL-1α or ₘaₕIL-1α b + ₕaₘIL-1α b have reduced metastatic burden. In both breast and prostate tumor models, mice administered ₙaₘIL-1α b showed no apparent reduction in metastasis at time of sacrifice. See Table 2.

**Table 2. Control of xenotransplanted tumors by treatment with aIL-1α antibody.**

| DIMDA-MB436 | PBS | ₘaₕIL-1α + ₕaₘIL-1α | ₘaₕIL-1α | ₕaₘIL-1α |
|---|---|---|---|---|
| Ascites | 6(100) | 0(0) | 1(17) | 4(66) |
| Lymph node | 6(100) | 1(17) | 2(33) | 6(100) |
| Peritoneal | 6(100) | 0(0) | 1(17) | 6(100) |
| Liver | 6(100) | 1(17) | 3(50) | 6(100) |
| | | | | |

| EstMDA-MB436 | PBS | ₘaₕIL-1α+ₕaₘIL-1α | ₘaₕIL-1α | ₕaₘIL-1α |
|---|---|---|---|---|
| Ascites | 6(100) | 0(0) | 0(0) | 4(66) |
| Lymph node | 6(100) | 4(66) | 5(83) | 6(100) |
| Peritoneal | 6(100) | 3(50) | 5(83) | 6(100) |
| Liver | 6(100) | 3(50) | 5(83) | 6(100) |
| | | | | |

| DIPC-3 | PBS | ₘaₕIL-1α + ₕaₘIL-1α | ₘaₕIL-1α | ₕaₘIL-1α |
|---|---|---|---|---|
| Ascites | 6(100) | 0(0) | 0(0) | 3(50) |
| Lymph node | 6(100) | 0(0) | 3(33) | 5(83) |
| Peritoncal | 6(100) | 1(17) | 2(33) | 6(100) |
| Liver | 6(100) | 0(0) | 1(17) | 6(100) |
| | | | | |

| EstPC3 | PBS | ₘaₕIL-1α+ₕaₘIL-1α | ₘaₕIL-1α | ₕaₘIL-1α |
|---|---|---|---|---|
| Ascites | 6(100) | 0 - | 0 | 5(83) |
| Lymph node | 6(100) | 3(50) | 3(50) | 6(100) |
| Peritoneal | 6(100) | 4(66) | 2(33) | 6(100) |
| Liver | 6(100) | 3(50) | 2(33) | 6(100) |

All PBS-treated mice are sacrificed by Day 50, whereas no aIL-1α-treated mice succumb to either breast or prostate xenotransplanted tumors after 60 days. aIL-1α treatment targeting tumor expressed IL-1α or endogenous (mainly leukocyte) derived IL-1α reduces metastatic burden in mice bearing either breast or prostate xenotransplanted cancer. aIL-1α treatment targeting tumor expressed IL-1α has a statistically more potent anti-tumor effect, whereas combined anti-tumor and anti-endogenous aIL-1α antibody treatment provides an almost complete block of metastatic tumor growth in nu/nu mice bearing either breast or prostate tumors. See Figure 1.

Animals receiving either ₘaₕIL-1α or ₕaₘIL-1α or combination of the two antibodies have reduced severity of clinical course of disease. All control animals eventually appear moribund and require sacrifice prior to the end of the study. In contrast, the animals receiving ₘaₕIL-1α + ₕaₘIL-1α are unexceptional in appearance, are well-groomed, active, show no signs of distress, exhibit normal growth and weight gain, and all survive for the duration of the experiment. Mice receiving the antibody combination do, however, reveal metastatic lesions observable after careful postmortem survey of organs. This is particularly evident in animals that have established metastatic tumors before beginning treatment. It appears that mice that receive treatment only after established metastatic lesions develop are unable to subsequently clear all the established lesions. However, metastatic lesions in these treated mice are apparently arrested.

This effect can be analyzed after inoculating mice with breast or prostate tumor and sacrificing the animals at time of detectable metastatic disease, which is the same course of disease where animals received treatment The abundance and size of metastatic lesions is noticeably greater in these mice than what is observed postmortem in animals receiving the ₘaₕIL-1α + ₕaₘIL-1α combination. It appears that the antibody treatment results in regression of the established metastatic disease.

The ₘaₕIL-1α + ₕaₘIL-1α treated mice that receive antibody injection starting Day 1 after tumor inoculation are almost completely prevented from developing metastasis. Only a single animal (17%) in each of the breast or prostate tumor group develops a metastatic lesion.

Xenotransplanted human tumors are used in a nude mouse model of tumor metastasis. The use of human tumors, expressing human IL-1α, allows us to attempt to treat mice by targeting either: human IL-1α expressed on tumors; murine IL-1α expressed on leukocytes (which for the sake of simplicity we shall refer to as endogenous IL-1α production); or by administering two different antibodies, simultaneously targeting both endogenous and tumor-derived IL-1α. This allows us to begin to disentangle, somewhat, the role in metastasis of tumor-derived IL-1α from that of endogenous produced IL-1α (expressed from leukocytic infiltrate or from tissues of the tumor microenvironment).

Results suggest that both endogenous and tumor-derived IL-1α play a role in tumor metastasis, because antibody directed against either source of IL-1α each improves survival in mice. Antibody directed against endogenous IL-1α is, however, considerably less effective at providing long-term survival benefit and does not protect mice from metastasis compared with antibody targeting tumor-derived IL-1α. Evidently, IL-1α expression from the tumors themselves is sufficient to promote metastasis in these models.

Antibody directed against IL-1α expressed by the tumor has potent anti-tumor effects. The profound anti-tumor effects in animals receiving anti-tumor-IL-1α antibody appears to involve a physiological blockade of IL-1α in tumor metastasis, but may also involve direct tumoricidal action of the antibody. We expect that targeting tumor-expressed IL-1α using an IgG1 antibody, an antibody subclass that efficiently induces complement fixation and antibody directed cellular cytotoxicity (ADCC), may represent a considerable tumoricidal action against the IL-1α expressing tumors in the nude mouse model. However, if the anti-tumor effect of antibody directed against tumor-derived IL-1α acts exclusively via an ADCC or other cytotoxic mechanism, there would not be an expectation for synergistic effect with the two antibodies. Nor would it be expected that antibody directed against endogenous IL-1α would impact survival; whereas, there is consistent, albeit modest, survival benefit seen in animals treated with antibody directed against endogenous IL-1α. It is possible that the survival benefit seen from targeting anti-endogenous IL-1α with the ₕaₘIL-1α antibody is a result of ₕaₘIL-1α crossreactivity with human IL-1α, thereby targeting tumor directly. We examined crossreactivity for the anti-murine IL-1α antibody, to assess whether the survival benefit is a direct result of crossreactivity of the antibody with tumor expressed IL-1α, inducing ADCC of the tumor, physiological IL-1α blockade of the tumor IL-1α production, or both. There is no apparent crossreactivity with the antibody. Furthermore, the ₕaₘIL-1α antibody does not efficiently bind murine Fc receptors and would not likely induce an effective ADCC response.

From the results of differential targeting of endogenous and tumor-expressed IL-1α in a xenotransplant model in nude mice we conclude that physiological blockade of IL-1α can reduce the lethality of tumors. The synergistic effect of survival benefit for the anti-IL-1α antibody combination, directed at both endogenous and tumor-derived IL-1α, provides compelling evidence that both tumor and endogenous sources of IL-1α are important in tumor metastasis in this model. These results also shed favorable light on other reports that suggest IL-1α plays a physiological role in the dynamic interplay between tumor and host; and that IL-1α expression can enhance metastatic potential of tumors.

Targeting IL-1α production by tumor cells using a monoclonal antibody is an effective means of prolonging survival and reducing metastatic burden. Antibody targeting of IL-1α expressing tumors would be of potential therapeutic value in human disease setting and may represent and effective therapeutic target for numerous forms cancer either early or advanced stages of disease.

It has been reported elsewhere that as much as 20% of persons analyzed have IL-1α-neutralizing autoantibody present in their sera. Moreover, these persons are reportedly healthy during lengthy observation periods. Similarly, IL-1α knockout mice are without an apparent phenotype (Horai et al. J. Exp. Med. 1998 187:1463-1475). Finally, it has been reported elsewhere (Svenson et al.) that animals can be simply and efficiently immunized with IL-1α to induce potent, neutralizing antibody responses against the cytokine. These findings suggest that an active immunotherapy, such as an immunization with IL-1α to induce neutralizing autoantibody, might also be an effective means of treating IL-1α expressing human cancer.

### EXAMPLE 5

### Materials and Methods

### Measurement of anti-IL-1α antibody titers by ELISA

Human or murine IL-1α, respectively, are incubated on 96 well ELISA plates over night, using 0.5µg/ml with a volume of 100µl per well. The plates are then washed 4 times with phosphate buffered saline (PBS) + 0,05% Tween 20, then saturated with a blocking solution containing 1% bovine serum albumin (BSA) in PBS + 0.05% Tween 20. 200µl of this blocking buffer are used per well for 1-2 hours at room temperature. Then plates are washed again 4 times with PBS + 0.05% Tween 20 (PBST). 100ml of serially diluted serum samples (1:2 dilutions in PBST + 1%BSA) are then added and incubated for one hour at room temperature or at 4°C over night. Then plates are washed again 4 times with PBST. Horseradish peroxidise (HRP) coupled anti-Fc antibody is then added as a secondary antibody (dilute 1:2000 in PBST with 1% BSA in, 100µl per well, 1hour, room temperature). Human: 0.2µl goat anti human IgG-HRP in 400µl PBST + 1%BSA. Mouse: 0.5µl HRP goat anti mouse IgG (H+L). Then plates are washed again 4 times with PBST. The colouring reaction is made with ABTS buffer (3-ethylbenzthiazoline-6-sulfonic acid, Sigma Cat. No. A-1888, 150 mg, 0.1 M citric acid, Fisher anhydrous, Cat. No. A-940, 500 ml, Adjust pH to 4.35 with NaOH pellets, Aliquot at 11 ml per vial and store at -20°C, 40% SDS (80 g SDS in 200 ml dd H₂O), Add 200 ml DMF (N.N-dimethyl formamide)). 100µl of the ABTS buffer are added to each well. The reaction is stopped by adding 100µl of 2% oxalic acid solution when good contrast is visible. The optical density is then measured with an ELISA reader at a wavelength of 405nm.

### Monitoring of animals during tumor challenge

Animal health was recorded by monitoring appearance, food and water intake, natural behaviour as well as provoked behaviour using the following scoring system: Score 0: no deviation form normal; Score 1: mild deviation from normal; Score 2: moderate deviation from normal; Score 3: substantial deviation from normal. If 3 is scored more than once, an extra 1 is given to each, making a maximum score of 15. Score 0-3: Normal. Score 4-7: Monitor carefully. Score 8-15: The animal is suffering. The animal is euthanized. The animals were also euthanized when they had a body weight loss of more than 15% or the body temperature dropped more than 3.0°C.

### Tumor cell lines

EL-4 cells were obtained from the American Type Culture Collection (ATCC, Manassas VA, USA). EL-4 was established from a lymphoma induced in a C57BL/6 mouse by 9,10-dimethyl-1,2-benzanthracene. Cells were cultured in Dulbecco's modified Eagle's medium with 4 mM L-glutamine adjusted to contain 1.5 g/L sodium bicarbonate and 4.5 g/L glucose, 90%; fetal calf serum, 10%.

PC-3 cells were obtained from American Type Culture Collection (ATCC, Manassas VA, USA). The PC-3 cells line was initiated from a bone metastasis of a grade IV prostatic adenocarcinoma from a 62-year-old male Caucasian. The cell line was grown using Ham's F12K medium with 2 mM L-glutamine adjusted to contain 1.5 g/L sodium bicarbonate, 90%; fetal bovine serum, 10%.

### Immunization of mice with IL-1a and IL-1b conjugated with PPD

IL-1α and IL-1β were obtained from eBioscience (San Diego, CA). PPD was obtained from the Statens Serum Institute (Copenhagen, Denmark). The method for conjugation was adapted from Svenson et al. (Svenson M. 2000). IL-1α or IL-1b were incubated for 48 h at 4°C with PPD at a ratio of 0.41 (w/w) and in the presence of 0.1% glutaraldehyde (IL-1/PPD = 0.41). As a control PPD was treated in parallel but without IL-1α or IL-1β. The cohjugate was then adsorbed to Al(OH)₃ (Rehydragel; Reheis Chemical, Dublin, Ireland) so that there was 1.5% Al(OH)₃ in the final volume. Incubation with Alum was for 90min at room temperature. The particles were then washed with 0.9% NaCl and resuspended it in 0.9% NaCl at 11 µg IL-1α/100µl suspension, assuming a 70% adsorption of IL-1α to Al(OH)₃ (found in pilot studies using ¹²⁵I-IL-1α). The IL-1β conjugate was prepared the same way. Control suspensions were diluted identically to match the amount of PPD in the IL-1α-PPD conjugate. The conjugates were stored at 4°C until use.

### EXAMPLE 6

### Generation of an anti-IL-1α antibody response in C57BL/6 mice

As the immune system is tolerant against self-proteins such as cytokines, however, such active vaccination has to break self tolerance. In case of most self proteins immune tolerance is caused by a lack of specific T cells as a consequence of negative selection in the thymus. In contrast, potentially self-reactive B cells are usually present. When injecting the self-protein like IL-1α alone, these B cells do not respond, due to the lack of T cell help. Coupling a foreign protein such as PPD to the self antigen IL-1α, T cell help for the B cell stimulation is provided, because the T cells recognize PPD which results in antibody production of stimulated B cells against IL-1α and PPD. Therefore, we vaccinated mice with an IL-1α-PPD conjugate in alum to ensure effective T-cell help for the IL-1β specific B-cells. Antibody titers were determined by ELISA. Groups of 5 mice received subcutaneous immunizations with 15µg of recombinant IL-1α conjugated to 10 µg-PPD using an incubation step with glutaraldehyde. The IL-1α-PPD conjugate is then absorbed to alum. Mice received three such subcutaneous immunizations with 2 weeks time interval.

Immunized mice produced high titers of anti-IL-1α antibodies, whereas the control mice immunized with PPD in alum failed to induce detectable antibody titers (Fig. 2). Induction of anti-IL-1α antibodies required at least 2 injections. After only one injection of recombinant IL-1α-PPD conjugate in alum no antibody response was detected in sera But after a third injection of recombinant IL-1α-PPD conjugate in alum all vaccinated mice produced anti-IL-1α antibodies.

### EXAMPLE 7

### Active immunization against IL-1α protects mice against tumor challenge with EL-4

C57BL/6 mice were actively immunized by three injections of 15µg murine IL-1α conjugated with 10 µg PPD in alum on days 0, 14 and 28 by subcutaneous administration in the neck region. The injection volume was 100 µl, and the amount of aluminium hydroxide was approx. 1mg. Control mice were treated similarly but with a preparation that contained the same amount of PPD and aluminium hydroxide but that did not contain IL-1α. Blood was sampled from the tail vain on days 0, 28, 42, and 56 in order to confirm the formation of anti-IL1α antibody responses by ELISA. On day 56 after the first immunization, all mice received an inoculum of 1,000 EL-4 lymphoma cells. Subsequently, mice were observed daily during the following four weeks. At the first onset of signs of sickness, mice were euthanized for macroscopic and histological quantification of tumor growth and metastasis.

Within 30 days after tumor challenge, control mice showed signs of sickness due to tumor progression, as evidenced by disseminated macroscopically visible metastasis in visceral organs. In contrast, none of the mice actively immunized against IL-1α showed clinical signs of disease.

### EXAMPLE 8

### Passive immunization against IL-1α protects against mouse lymphoma EL-4

C57BL/6 mice were actively immunized against IL-1α with 3 subcutaneous injections of IL-1α-PPD conjugate in alum. After 56 days their serum was collected and generation of anti-IL-1α autoantibody titers were confirmed by ELISA. 200µl of such serum was passively transferred to 6 weeks old C57BL/6 mice. These passive serum transfers were repeated every week. Control C57BL/6 mice received 200µl of serum from naive C57BL/6 mice with weekly intervals. Together with the first serum transfer all mice received an inoculum of 1,000 EL-4 lymphoma cells. Subsequently, mice were observed daily during the following four weeks. At the first onset of signs of sickness, mice were euthanized for macroscopic and histological quantification of tumor growth and metastasis.

Within 30 days, control mice succumbed to the tumor, as evidenced by disseminated macroscopically visible metastasis in visceral organs. In contrast, none of the mice receiving the passive serum transfer with polyclonal anti-IL-1α antiserum showed clinical signs of disease.

### EXAMPLE 9

### Passive immunization against IL-1a protects SCID mice against PC-3 xenografted tumor

C57BL/6 mice were actively immunized against IL-1α with 3 subcutaneous injections of IL-1α-PPD conjugate in alum. After 56 days their serum was collected and generation of anti-IL-1α autoantibody titers were confirmed by ELISA. 200 µl of such serum was passively transferred to 6 weeks old female SCID mice. These passive serum transfers were repeated every week. Control SCID mice received 200 µl of serum from naive C57BL/6 mice with weekly intervals. Together with the first serum transfer all mice received a subcutaneous inoculum of 10⁷ PC-3 cells into the flanks. Mice with palpable tumors were identified every week.

Within 30 days, control mice had developed palpable tumors at the site of inoculation, whereas none of the mice receiving the passive serum transfer with polyclonal anti-IL-1α antiserum developed a palpable tumor.

### EXAMPLE 10

### ADCK-Antibody dependent complement mediated killing

C57BL/6 mice were actively immunized against IL-1α with 3 subcutaneous injections of IL-1α-PPD conjugate in alum. After 56 days their serum was collected and generation of anti-IL-1α autoantibody titers were confirmed by ELISA. Sera were heat inactivated. 50 µl of an EL-4 cell suspensions were plated into 96 well plates. To each of these wells 15 µl of 1:2 serial dilutions of the heat inactivated serum was added. Plates were then incubated for 20 minutes at 37°C. Then 25ml of murine serum were added to each well. After another 5h incubation at 37°C wells are photographed and then the cells counted in a counting chamber using trypan blue to distinguish dead from alive cells.

The polyclonal mouse-anti-mouseIL-1α antiserum mediated complement dependent killing of EL-4 tumor cells in a concentration dependent fashion. See Figure 3.

## Claims

1. Anti-IL-1α antibodies for use in treating cancer in a mammalian patient.

2. The anti-IL-1α antibodies of claim 1 wherein the patient is a human.

3. The anti-IL-1α antibodies of claim 1 wherein the patient is selected from the group consisting of a mouse, a pig, a goat, a dog, a cat, and a sheep.

4. The anti-IL-1α antibodies of claim 1 wherein the cancer is metastatic.

5. The anti-IL-1α antibodies of claim 1 wherein the cancer is breast or prostate cancer.

6. The anti-IL-1α antibodies of claim 1 wherein the antibodies are polyclonal.

7. The anti-IL-1α antibodies of claim 1 wherein the antibodies are monoclonal.

8. The anti-IL-1α antibodies of claim 1 wherein the antibodies are selected from the group consisting of Fab, F(ab')2, scFv, Fv, diabodies, minibodies, Kappa bodies, and Janusins.

9. IL-1α for use in treating cancer, whereby the patient generates IL-1α autoantibodies.

10. The 1L-1α of claim 9 wherein the patient is a human.

## Patentansprüche

1. Anti-IL-1α-Antikörper zur Verwerdung für das Behandeln von Krebserkrankungen in einem Säugetierpatienten.

2. Anti-IL-1α-Antikörper nach Anspruch 1, wobei der Patient ein Mensch ist.

3. Anti-IL-1α-Antikörper nach Anspruch 1, wobei der Patient ausgewählt ist aus der Gruppe bestehend aus einer Maus, einem Schwein, einer Ziege, einem Hund, einer Katze, und einem Schaf.

4. Anti-IL-1α-Antikörper nach Anspruch 1, wobei die Krebserkrankung metastasierend ist.

5. Anti-IL-1α-Antikörper nach Anspruch 1, wobei die Krebserkrankung Brust- oder Prostatakrebs ist.

6. Anti-IL-1α-Antikörper nach Anspruch 1, wobei die Antikörper polyklonal sind.

7. Anti-IL-1α-Antikörper nach Anspruch 1, wobei die Antikörper monoklonal sind.

8. Anti-IL-1α-Antikörper nach Anspruch 1, wobei die Antikörper ausgewählt sind aus der Gruppe bestehend aus Fab, F(ab')2, scFv, Fv, Diabodies, Minibodies, Kappabodies, und Janusine.

9. IL-1α zur Verwendung für das Behandeln von Krebserkrankungen, wobei der Patient IL-1α-Autoantikörper bildet.

10. IL-1α nach Anspruch 9, wobei der Patient ein Mensch ist.

## Revendications

1. Anticorps anti-IL-1α destinés à être utilisés dans le traitement du cancer chez un patient mammifère.

2. Anticorps anti-IL-1α selon 1α revendication 1 où le patient est un humain.

3. Anticorps anti-IL-1α selon 1α revendication 1 où le patient est choisi dans le groupe consistant en une souris, un porc, une chèvre, un chien, un chat et un mouton.

4. Anticorps anti-IL-1α selon 1a revendication 1 où le cancer est métastatique.

5. Anticorps anti-IL-1α, selon la revendication 1 où le cancer est le cancer du sein ou de la prostate.

6. Anticorps anti-IL-1a selon la revendication 1 où les anticorps sont polyclonaux.

7. Anticorps anti-IL-1α selon la revendication 1 où les anticorps sont monoclonaux.

8. Anticorps anti-IL-1α selon la revendication 1 où les anticorps sont choisis dans le groupe consistant en Fab, F(ab')2, scFv, diabodies, minibodies, kappa bodies et Janusins.

9. IL-1α, destinée à être utilisée dans le traitement du cancer, de sorte que le patient produit des autoanticorps anti-IL-1α.

10. IL-1α selon la revendication 9 où le patient est un humain.
